# EUROPÄISCHE PATENTSCHRIFT

(11) **EP 2 981 508 B1**
(45) Veröffentlichungstag und Bekanntmachung des Hinweises auf die Patenterteilung: **09.09.2020**
(21) Anmeldenummer: 14717708.3
(22) Anmeldetag: 03.04.2014
(51) Int. Cl.: C02F 1/28, C02F 101/36, B01J 20/22

(54) **VERFAHREN ZUR ABTRENNUNG FLUORIERTER ORGANISCHER VERBINDUNGEN AUS KONTAMINIERTEN FLUIDEN UND HIERFÜR VERWENDETE ADSORBENSKOMPONENTE UND ADSORBENS-KIT**
METHOD FOR REMOVING FLUORINATED ORGANIC COMPOUNDS FROM CONTAMINATED FLUIDS, AND ADSORBENT COMPONENT AND ADSORBENT KIT USED THEREFOR
PROCÉDÉ DE SÉPARATION DE COMPOSÉS ORGANIQUES FLUORÉS PRÉSENTS DANS DES FLUIDES CONTAMINÉS, ÉLÉMENTS ADSORBANTS UTILISÉS À CETTE FIN ET KIT D'ÉLÉMENTS ADSORBANTS

(30) Priorität: 05.04.2013 DE 102013206066
(43) Veröffentlichungstag der Anmeldung: 10.02.2016
(73) Patentinhaber: Fraunhofer-Gesellschaft zur Förderung der angewandten Forschung e.V., 80686 München (DE); Cornelsen Solutions GmbH, 45141 Essen (DE)
(72) Erfinder: BRUZZANO, Stefano, 47051 Duisburg (DE); CORNELSEN, Martin, 44795 Bochum (DE)
(86) Internationale Anmeldenummer: PCT/EP2014/056761
(87) Internationale Veröffentlichungsnummer: WO 2014/161973

(56) Entgegenhaltungen:
- WO-A1-2011/069189

## Beschreibung

Die Anmeldung betrifft ein Verfahren, das insbesondere zur Abtrennung fluorierter organischer Verbindungen aus Fluiden geeignet ist, ein hierfür einsetzbares Kit und eine hierfür einsetzbare, insbesondere lösliche, erste Adsorbenskomponente mit einer lipophilen Gruppe und einer hydrophilen Gruppe. Hierbei erfolgt zum einen eine Kontaktierung des kontaminierten Fluid zumindest mit der ersten, im Regelfall amphiphilen, Adsorbenskomponente. Anschließend kann eine Kontaktierung mit einer zweiten, festen Adsorbenskomponente erfolgen, die eine Adduktbildung ermöglicht, so dass das beladene Adsorbens vom gereinigten Fluid abgetrennt werden kann.

Fluorierte organische Verbindungen besitzen ausgezeichnete und einzigartige technologische Eigenschaften, weshalb sie seit langem in vielen unterschiedlichen industriellen Produkten und Prozessen Verwendung finden (z.B. als Textil- und Papierausrüstungen, als wasser- und schmutzabweisende Beschichtungen, als Hydraulikflüssigkeiten, als Feuerlöschschäume, in der Teflonherstellung sowie in der Galvanik). Aufgrund ihrer spezifischen Eigenschaften sind diese Substanzen für viele Anwendungen oft alternativlos. Allerdings weisen fluorierte Verbindungen, insbesondere perfluorierte Tenside, ein ökotoxisches sowie ein humantoxisches Potential auf, das u.a. auf der hohen Tendenz zur Bioakkumulation beruht. Aufgrund ihrer hohen Persistenz sind perfluorierte Tenside seit einigen Jahren ubiquitär in der Umwelt nachweisbar. Sie gelangen im Wesentlichen durch Abwässer aus fluorochemischen Produktionsverfahren oder durch die Nutzung und Entsorgung der entsprechenden Produkte in die Umwelt. Beispielsweise sind hier perfluorierte Tenside als Schaumbildner in Feuerlöschschäumen sowie Netz- und Antischleiermittel in Betrieben der Galvanik, Fototechnik oder Halbleitertechnik zu nennen.

In den chemikalienrechtlichen Vorschriften und Richtlinien vieler Länder, z.B. der europäischen Richtlinie 2006/122/EG wurde auf die Gefahren von Perfluoroctansäure und Perfluoralkylsulfonsäuren aufmerksam gemacht und deren Verwendung stark eingeschränkt.

In einer Reihe von Industriebetrieben sind perfluorierte Tenside allerdings unverzichtbar, insbesondere in der Galvanik, im Brandschutz sowie in der Foto- und Halbleitertechnik; daher wurden Ausnahmetatbestände für diese Anwendungszwecke in den jüngsten gesetzlichen Regelungen aufgenommen. Beispielsweise werden diese Stoffe in der Galvanotechnik im Arbeitsschutz eingesetzt (Antischleier- und Netzmittel); zur Immobilisierung der sehr giftigen und krebserzeugenden Chromsäure werden sie als Schaumsperre bzw. Sprühnebelverhinderer verwendet.

Um einen Eintrag von fluorierten organischen Verbindungen, insbesondere hochtoxischen Verbindungen wie speziellen perfluorierten Tensiden (PFT) in Abwasser und Grundwasser zu reduzieren, werden heute verschiedene Maßnahmen in den Betrieben umgesetzt. Insbesondere ist die Abtrennung und getrennte Entsorgung der mit diesen Stoffen belasteten Abwasserteilströme zu nennen. Von entscheidender Bedeutung ist hierfür der Einsatz unterschiedlicher Behandlungsverfahren der Abwasser- und Grundwasserreinigung.

Bei der Reinigung besteht allerdings das Problem, dass insbesondere perfluorierte Tenside aufgrund ihrer chemisch-physikalischen Eigenschaften weder mit gängigen biologischen Verfahren noch durch klassische physikalische Methoden wie Strippen aus kontaminierten Fluiden wirksam abgetrennt werden können.

Die US 2007/0138110 A1 beschreibt beispielsweise die Entfernung perfluorierter Tenside mittels kapazitiver Deionisation kontaminierter Wässer. Dieses Verfahren ist allerdings für die Reinigung großer Abwassermengen bezüglich Anschaffung, Betrieb und Wartung der Anlagen zu kostenintensiv. Gleichermaßen gilt dies auch für nassoxidative Methoden, z.B. mittels UV-, Ozon-, Peroxiden- oder Mikrowellenstrahlung.

Bei einigen physikalischen Abtrennverfahren wie der Membrantechnik liegen nach der Behandlung hochkonzentrierte wässrige Lösungen als Abfälle vor, deren (thermische) Entsorgung schwierig und somit unwirtschaftlich ist.

Unter den chemischen Abtrennverfahren sind insbesondere Adsorptionsmethoden zu nennen. Zur Reinigung von Abwässern werden beispielsweise Adsorberharze eingesetzt. Sie zeigen ein sehr selektives Adsorptionsvermögen und ein große Adsorptionskapazität im Bereich hoher Schadstoffkonzentrationen. Beispielsweise offenbart die WO 2008/066748 A1 die Verwendung von Ionenaustauschermaterialien. Trotzdem konnten sich Adsorberharze als Adsorptive zur Aufbereitung von Abwässern und Grundwässern nicht durchsetzen. Sie sind im Vergleich zu Aktivkohle teurer und in der Regenerierung aufwändiger.

Daher wird zur Reinigung von PFT-haltigen Abwässern vielfach die Adsorption an Aktivkohle angewendet. Allerdings können fluorierte Tenside, insbesondere kurzkettige perfluorierte Tenside, hiermit nicht selektiv abgetrennt werden.

Beispielsweise offenbart die EP 1 561 729 A1 ein Adsorptionsverfahren auf Basis von Aktivkohle, um fluorierte Emulgatoren in einem Abwasser, das Partikel aus Fluorpolymeren enthält, abzutrennen. Dazu werden dem Abwasser vor der Aktivkohleadsorption nicht-ionische oder anionische Tenside zugegeben, um die Ablagerung von Polymerpartikeln auf der Aktivkohle und somit ein Zusetzen des Filters zu verhindern.

Ferner offenbart die WO 2011/069189 A1 unter anderem die Abtrennung von fluorierten Verbindungen aus verunreinigten Flüssigkeiten mittels organisch-modifizierter Adsorbenzien auf Basis von Palygorskit-Ton. Zur chemischen Modifikation der Tone werden Fettsäure-Amine eingesetzt.

Es ist somit eine Aufgabe der vorliegenden Erfindung, die Nachteile des Stands der Technik zu überwinden und ein Adsorptionsmittel und ein Verfahren zur Anwendung von Additiven für die Abtrennung fluorierter organischer Verbindungen, insbesondere fluorierter oder perfluorierter Tenside, aus kontaminierten Fluiden anzugeben, das die Nachteile des Stands der Technik überwindet.

Diese Aufgaben werden durch den Gegenstand der unabhängigen Ansprüche gelöst. Weitere Ausgestaltungen und Weiterbildungen sind Gegenstand von Unteransprüchen und gehen weiterhin auch aus der nachfolgenden Beschreibung hervor.

Erfindungsgemäß wurde erkannt, dass fluorierte organische Verbindungen aus Fluiden zumindest mittels eines Kits, das eine erste Adsorbenskomponente und eine zweite Adsorbenskomponente umfasst, abgetrennt werden können, insbesondere betrifft dies die Abtrennung perfluorierter organischer Verbindungen und/oder fluorierter Tenside aus hiermit kontaminierten Fluiden. Bei einigen Ausführungsformen reicht aber sogar die Verwendung nur der ersten Adsorbenskomponente zur Abtrennung dieser Stoffe aus.

Die erste Adsorbenskomponente ist eine chemische Verbindung, die eine lipophile Gruppe und eine hydrophile Gruppe enthält (und damit eine amphiphile chemische Verbindung), wobei die hydrophile Gruppe zumindest eine kationische Gruppe enthält.

Die chemische Verbindung, die eine lipophile Gruppe und eine hydrophile Gruppe enthält, kann unter Normalbedingungen als Flüssigkeit oder auch in fester Form vorliegen und für die Verwendung des erfindungsgemäßen Verfahrens (beispielsweise mittels eines Lösungsvermittlers) in eine gelöste Form gebracht werden, hierfür reicht auch die Ausbildung einer Lösung in dem zu reinigenden Fluid aus. Unter einer gelösten Form wird hierbei auch verstanden, dass zumindest ein Gewichtsanteil der ersten Adsorbenskomponente, der 10% des Gewichtsanteils der im kontaminierten Fluid vorliegenden fluorierten organischen Verbindungen entspricht, im kontaminierten Fluid in gelöster Form vorliegt. Häufig wird aber eine vollständige Auflösung erfolgen. Zu einem Vorliegen in gelöster Form werden erfindungsgemäß stets auch im Fluid gebildete Mizellen gezählt. Die Löslichkeit kann daher in einfacher Weise über die Bestimmung der Menge nichtaufgelöster erster Adsorbenskomponente ermittelt werden. Im Fall einer Lösung für das erfindungsgemäße Verfahren dann also eine erste Adsorbenskomponente verwendet werden, bei der die amphiphile chemische Verbindung in einem Lösungsmittel gelöst vorliegt oder in einem zu reinigenden beispielsweise wässrigen Fluid aufgelöst werden kann.

Unter einer lipophilen Gruppe in der ersten Adsorbenskomponente wird verstanden, dass als Struktur zumindest eine der folgenden Gruppen enthalten ist: eine Alkyl-Gruppe, die zumindest eine Octyleneinheit (also eine Einheit der Formel -C₈H₁₆-, d.h. einen Octylrest oder eine nicht terminale Alkylgruppe, beispielsweise eine mit der hydrophilen Gruppe substituierte Octylengruppe) umfasst, oder alternativ eine Aryl-Gruppe oder eine Aralkyl-Gruppe.

Durch den spezifischen Strukturaufbau der ersten Adsorbenskomponente kann diese gewissermaßen als Carrier-Verbindung fungieren; für die abzutrennenden Stoffe kann eine maßgeschneiderte Einstellung der Wechselwirkungen Additiv/ fluorierte organische Verbindung erfolgen, sodass die Bildung eines Adduktes erfolgt, das mittels der zweiten Adsorbenskomponente nachfolgend abgetrennt werden kann oder bereits ohne Zusatz der zweiten Adsorbenskomponente aus dem zu reinigenden Fluid ausfällt (wobei unter Ausfällung erfindungsgemäß neben Sedimentation insbesondere auch Ausflockung und Flotation verstanden wird). Für die erste Adsorbenskomponente kann auf eine Palette einfacher Ausgangsmaterialien beispielsweise kommerziell erhältliche Tensidstrukturen zurückgegriffen werden, die dem Fachmann aus der Chemie im Allgemeinen und den Bereichen wie Waschmittel-, Papier-, Lebensmittel- und Wasserchemie im Speziellen bekannt sind.

Bei der optionalen zweiten Adsorbenskomponente handelt es sich um ein herkömmliches festes Adsorbens, also um einen Stoff, der aufgrund seiner großen, aktiven Oberfläche befähigt ist, abzutrennende chemische Verbindungen aus gasförmigen oder flüssigen Mischungen an seiner Grenzfläche selektiv anzureichern (das heißt zu adsorbieren). Die zweite Adsorbenskomponente wird daher erfindungsgemäß auch häufig als "feste Adsorbenskomponente" bezeichnet. Insbesondere können als zweite Adsorbenskomponente Adsorbentien verwendet werden, die sich auch als stationäre Trennphase eignen und mit denen die Abtrennung fluorierter Verbindungen, insbesondere fluorierter Tenside prinzipiell erfolgen kann. Derartige Adsorbentien sind dem Fachmann seit langer Zeit bekannt. Erfindungsgemäß wurde beobachtet, dass die Wirkung der festen Adsorbenskomponente durch die erste Adsorbenskomponente ergänzt oder verstärkt wird.

Unter fest wird bei der festen Adsorbenskomponente verstanden, dass sich diese Komponente im kontaminierten Fluid nicht löst. Entsprechendes gilt auch für ein gegebenenfalls vorliegendes Lösungsmittel, das für die Überführung einer festen amphiphilen chemischen Verbindung in den flüssigen Zustand, das heißt für die Bereitstellung einer ersten Adsorbenskomponente erforderlich ist. Auch in einem derartigen Lösungsmittel löst sich die feste Adsorbenskomponente nicht.

Eine Lösung der vorstehenden Aufgabe erfolgt daher durch das folgende erfindungsgemäße Verfahren unter Verwendung der ersten Adsorbenskomponente :
Nachfolgend erfolgt in Schritt B) die Kontaktierung des kontaminierten, in Form einer wässrigen Flüssigkeit vorliegenden Fluids mit der ersten Adsorbenskomponente, wobei die erste Adsorbenskomponente während der Kontaktierung zumindest teilweise in gelöster Form vorliegt, wobei durch die Kontaktierung ein Addukt zwischen der abzutrennenden fluorierten organischen Verbindung und der in gelöster Form vorliegenden ersten Adsorbenskomponente gebildet wird. Schließlich wird in einem Schritt D) die mindestens erste Adsorbenskomponente mit den adsorbierten fluorierten organischen Verbindungen von dem Fluid abgetrennt.

Erfindungsgemäß wurde festgestellt, dass mittels eines derartigen Verfahrens durch das Kontaktieren eines verunreinigten Fluids mit einer Carrierverbindung, nämlich der ersten Adsorbenskomponente, eine sehr selektive Abtrennung fluorierter organischer Verbindungen, insbesondere fluorierter oder perfluorierter Tenside möglich ist. Ohne hierauf beschränkt werden zu wollen, wird davon ausgegangen, dass diese Beobachtung auf folgender Modellvorstellung beruht:
In Schritt B) erfolgt eine Wechselwirkung zwischen der abzutrennenden fluorierten organischen Verbindung und der ersten Adsorbenskomponente, wobei die Wechselwirkung zwischen den beiden Stoffen so stark ist, dass man die erste Adsorbenskomponente auch als Carrier-Verbindung bezeichnen kann. Wird die Konzentration der ersten Adsorbenskomponente im Fluid groß genug gewählt, so kann in vorteilhafter Weise auch eine Mizellenbildung der amphiphilen ersten Adsorbenskomponente erfolgen; die abzutrennende fluorierte organische Verbindung kann sich dann gegebenenfalls auch im Inneren der Mizelle anordnen. Die erste Adsorbenskomponente wird ferner so gewählt, dass Sie entweder aufgrund ihrer Löslichkeitseigenschaften durch Adduktbildung mit den abzutrennenden Stoffen aus dem Fluid ausfällt oder nach Adduktbildung durch Zusatz der zweiten Adsorbenskomponente ausgefällt wird.

In Schritt D) kann schließlich das Addukt aus amphiphiler erster Adsorbenskomponente und fluorierter organischer Verbindung oder im Fall der nachfolgend beschriebenen Ausführungsform mit zweiter Adsorbenskomponente das Addukt aus amphiphiler erster Adsorbenskomponente, zweiter Adsorbenskomponente und fluorierter organischer Verbindung aus dem kontaminierten Fluid abgetrennt werden. Dies ist beispielsweise mittels Sedimentation oder Filtration möglich.

Mit dem erfindungsgemäßen Verfahren, der ersten Adsorbenskomponente sowie dem Kit aus erster Adsorbenskomponente und zweiter Adsorbenskomponente wird nun eine Methode zur Verfügung gestellt, mit der kostengünstig fluorierte organische Verbindungen, insbesondere perfluorierte Tenside, aus kontaminierten Fluiden abgetrennt werden können. Durch Wechselwirkung der amphiphilen chemischen Verbindung über die lipohilen Gruppen einerseits und vorhandene kationische Ladung andererseits wird die Bildung eines Adduktes hervorgerufen, das entweder von selbst ausfällt, zumindest aber eine bessere Adsorption an der festen Adsorbenskomponente (beispielsweise Aktivkohle) zeigt als die reine fluorierten organischen Verbindung.

Ferner hat sich gezeigt, dass das erfindungsgemäße Verfahren nicht ausschließlich zur Abtrennung von perfluorierten oder teilfluorierten Tensiden geeignet ist, sondern auch für perfluorierte Verbindungen im Allgemeinen (die aufgrund der Wechselwirkung mit den hydrophoben Gruppen gut abgetrennt werden können) als auch von fluorierten anderen organischen Verbindungen (wie beispielsweise organischen Verbindungen mit fluorierten Alkylgruppen oder fluorierten aromatischen Verbindungen, die wiederum mittels der hydrophoben Gruppen leichter adsorbiert werden können). Zu nennen sind insbesondere Fluoralkylalkanole bzw. Fluoralkylalkandiole, Fluortelomere, Fluoroacrylate, Fluoromethacrylate, Fluoralkyldimethylbetaine, Fluoroalkylethylbetaine, perfluorierte und teilfluorierte Fettsäureester, perfluorierte und teilfluorierte Fettalkoholsulfate, perfluorierte und teilfluorierte Fettalkoholpolyglykolethersulfate, nicht-ionische polymere fluorierte Tenside, aliphatische teilfluorierte Polymerester und Perfluoroalkylbetaine.

Das das erfindungsgemäße Verfahren eignen sich ferner auch zur Abtrennung anderer unerwünschter Stoffe aus Fluiden, insbesondere anionischer Stoffe, die mit einer gegebenenfalls enthaltenen kationischen Gruppe wechselwirken und dadurch abgetrennt werden können. Zu nennen sind hierzu insbesondere Uranylverbindungen oder auch anorganische oder organische Arsenate und Antimonate.

Das erfindungsgemäße Verfahren, die erste Adsorbenskomponente und das erfindungsgemäße Kit eignen sich schließlich nicht nur zur Reinigung von kontaminierten Wässern sondern auch von anderen Flüssigkeiten, wie organischen Lösungsmitteln (sofern keine chemische Reaktion des Lösungsmittels mit Substituenten der ersten Adsorbenskomponente erfolgt). Ferner ist grundsätzlich auch die Reinigung von Gasen, insbesondere von Luft, möglich, allerdings gestaltet sich bei einem mehrstufigen Adsorptionsprozess die Verfahrensführung deutlich schwieriger als bei Flüssigkeiten. Gegebenenfalls kann die erste Adsorbenskomponente und das erfindungsgemäße Kit auch zur Anreicherung von Wertstoffen eingesetzt werden, beispielsweise besonders teurer fluorhaltiger Verbindungen; derartige Wertstoffe können in einem Desorptionsprozess nachfolgend wiedergewonnen werden.

Wie oben ausgeführt, enthält die erfindungsgemäß eingesetzte erste Adsorbenskomponente eine lipophile Gruppe. Diese umfasst entweder eine Alkyl-Gruppe, eine Aryl-Gruppe und/oder eine Aralkyl-Gruppe. Die Alkyl-Gruppe kann eine Octyleinheit sein oder eine Octyleneinheit enthalten. Mit noch längeren Alkylen- bzw. Alkyl-Gruppen kann die Lipophilie weiter erhöht werden, was bei bestimmten abzutrennenden fluorierten organischen Verbindungen von Vorteil sein kann. So kann als lipophile Gruppe beispielsweise eine Decyleneinheit oder Decyleinheit verwendet werden oder eine Dodecyleinheit bzw. Dodecyleneinheit. Als Aryl-Gruppen kommen insbesondere PhenylGruppen oder Phenylen-Gruppen in Betracht, die unsubstituiert sind oder auch substituiert sein können (auch hier insbesondere wiederum mit weiteren reinen Kohlenwasserstoffen in aromatischer oder aliphatischer Form). Als Aralkyl-Gruppen kommen beispielsweise Benzyl-Gruppen in Betracht. Die Aralkylgruppen, beispielsweise die Benzyl- bzw. Benzylen-Gruppen können substituiert oder unsubstituiert sein. Auch Benzylen-Gruppen, bei denen an den aromatischen Ring die ionische Gruppe gebunden ist, sind denkbar. Um die Wechselwirkung mit den zu adsorbierenden fluorhaltigen Verbindungen zu verstärken können die Alkyl-Gruppe, Aryl-Gruppe beziehungsweise Aralkyl-Gruppe ebenfalls teilfluoriert oder perfluoriert sein. Insbesondere sind hierbei teilfluorierte oder perfluorierte Alkyl- oder Alkyleneinheiten in diesen Gruppen zu nennen.

Um zu erreichen, dass die erste Adsorbenskomponente bereits durch Adduktbildung mit den abzutrennenden Stoffen aus dem Fluid ausfällt, sollte die erste Adsorbenskomponente insbesondere so gewählt sein, dass - abhängig von der abzutrennenden fluorhaltigen Verbindung - eine Addukt mit geringer Wasserlöslichkeit entsteht. Im Grundsatz wird dies bereits durch die genannten Alkyl-, Aralkyl- oder Aryl-Gruppen erreicht. Dieser Effekt kann aber noch verstärkt werden, wenn bereits die (bezogen auf die abzutrennenden fluorhaltigen Verbindungen) äquimolare Menge der ersten Adsorbenskomponente (auch bei Konzentrationen Kleiner als 1 µmol/l) ohne einen Lösungsvermittler nicht vollständig in dem Fluid löslich ist. Unabhängig hiervon eignen sich für die Verstärkung dieser Eigenschaften auch Verbindungen, die zumindest zwei Alkyl-, Aralkyl- oder Aryl-Gruppen gemäß der Definition des vorstehenden Absatzes und/oder einen Alkylrest mit zumindest einer Tetradecylen-Einheit aufweisen, insbesondere aber Verbindungen, die mindestens zwei Gruppen mit Tetradecylen-Einheiten enthalten.

Generell ist eine "kationische Gruppe" der ersten Adsorbenskomponente im Rahmen dieser Anmeldung so zu verstehen, dass diese Gruppe in einem gegebenen Fluid stets kationisch ist oder alternativ nur unter bestimmten Bedingungen als Kation vorliegt. Insbesondere bei wässrigen Fluiden kann die kationische Gruppe daher bei höheren pH-Werten in der Neutralform und nur bei niedrigeren pH-Werten zumindest teilweise in der protonierten Form vorliegen, wie dies häufig bei primären, sekundären oder tertiären Aminen der Fall ist. Wesentlich ist dann, dass auch in einem gegebenen kontaminierten Fluid die kationische Gruppe zu einem gewissen Anteil in der protonierten Form vorliegt. So weisen primäre, sekundäre und tertiäre niedermolekulare Amine in Wasser einen pk_{B} von ca. 4 auf. In pH neutralen Wässern liegt daher im Regelfall überwiegend die protonierte Form vor. Nur in stark alkalischen Wässern kann der protonierte Anteil deutlich niedriger sein. Er sollte aber zumindest 1 %, bevorzugt zumindest 10%, sein. Um zu einer effektiveren Adsorption zu gelangen, kann bei derartigen kationischen Gruppen dann eine Vorbehandlung der kontaminierten Wässer erfolgen, in der der pH-Wert gesenkt wird.

Erfindungsgemäß umfasst die in der ersten Adsorbenskomponente enthaltene kationische Gruppe ein Ammonium-Ion, meist ein organisch substituiertes Ammonium (wobei häufig die hydrophobe Gruppe als "organischer Substituent" des Ammoniums fungiert), ein primäres, sekundäres oder tertiäres Amin (Gegebenenfalls kann bei primären, sekundären oder tertiären Aminen in einer Modifizierungsreaktion eine Quaternisierung erfolgen, so dass zumindest teilweise quaternäre Amingruppen gebildet werden), ein organisch substituiertes Phosphonium (bei dem im Regelfall die hydrophobe Gruppe als organischer Substituent dient)und einen Metallkomplexe, beispielsweise einen Übergangsmetallkomplex (hierbei kann eine Verknüpfung mit der hydrophoben Gruppe ebenfalls im Metallkomplex vorhanden sein, wobei hierbei eine ganze Reihe von Möglichkeiten besteht; beispielsweise kann die hydrophobe Gruppe Bestandteil eines Liganden des Metallkomplexes sein oder auch der Ligand selbst sein). Im Einzelfall kann die kationische Gruppe auch in einer zwitterionischen Verbindung, beispielsweise einer Betain-Struktur vorliegen (insbesondere sind hier Carbo-Betaine und Sulfo-Betaine zu nennen und zwar mit einer Ammonium-Gruppe als Kation). Im Regelfall haben sich aber die rein kationischen, d.h. nicht zwitterionischen Strukturen als vorteilhafter erwiesen. Liegt als kationische Gruppe ein organisch substituiertes Ammonium oder Amin vor, so ist dabei häufig einer der Substituenten die lipophile Gruppe oder enthält die lipophile Gruppe. Als weitere organische Substituenten liegen häufig Alkyl-Gruppen vor, beispielsweise Methyl-, Ethyl- oder n- bzw. i-Propyl-Gruppen.

Mit derartigen kationischen Gruppen können unerwünschte Verbindungen wie Tenside, aber auch anionische anorganische Gruppen wie Uranyl-Gruppen besonders gut abgetrennt werden.

Gemäß einer weiteren Ausführungsform erfolgt die Abtrennung der fluorierten organischen Verbindungen aus kontaminierten Fluiden mittels eines Kits aus der ersten und einer zweiten Adsorbenskomponente. Es wird dann in Schritt A) zusätzlich die zweite Adsorbenskomponente bereitgestellt, die ein festes Adsorbens ist. Ferner umfasst das Verfahren dann den Schritt C), in dem die Kontaktierung des Fluids mit der zweiten Adsorbenskomponente stattfindet.

Erfindungsgemäß wurde festgestellt, dass mittels des Verfahrens nach dieser Ausführungsform, nämlich durch das Kombinieren der festen Adsorbenskomponente mit der ersten Adsorbenskomponente, eine noch selektivere Abtrennung fluorierter organischer Verbindungen, insbesondere fluorierter oder perfluorierter Tenside möglich ist. Bei ersten Adsorbenskomponenten, die nicht bereits als Addukt mit den fluorierten Verbindungen ausfallen, wird erst durch den Zusatz der zweiten Adsorbenskomponente eine Abtrennbarkeit erreicht.

In Schritt C) wird das kontaminierte Fluid mit der festen Adsorbenskomponente in Kontakt gebracht, so dass einerseits das gebildete Addukt aus amphiphiler erster Adsorbenskomponente und fluorierter organischer Verbindung und andererseits auch gegebenenfalls vorliegende Anteile nicht an die erste Adsorbenskomponente gebundener freier fluorierter organischer Verbindung auf der Oberfläche der zweiten Adsorbenskomponente gebunden werden können.

Gemäß einer weiteren Ausführungsform wird das erfindungsgemäße Verfahren so durchgeführt, dass Schritt B) und C) gleichzeitig durchgeführt werden oder, dass Schritt B) vor Schritt C) durchgeführt wird. Aus den oben beschriebenen theoretischen Grundlagen ergibt sich, dass im Regelfall zunächst eine Adsorption von abzutrennender fluorierter organischer Verbindung erfolgt und erst nachfolgend eine Adsorption von insbesondere nicht aus dem Fluid ausgefallenem Adsorptionsprodukt von Schritt C) an der festen Adsorbenskomponente. Insofern ergibt sich bereits aufgrund theoretischer Überlegungen, dass im Regelfall Schritt B) vor Schritt C) durchgeführt werden sollte. Eine Durchführung von Schritt C) nach Schritt B) ist weniger günstig, zumal das Risiko besteht, dass die erste Adsorbenskomponente bereits mit der zweiten Adsorbenskomponente in Wechselwirkung getreten ist und damit eine Wechselwirkung mit der abzutrennenden fluorierten organischer Verbindung erschwert wird.

Gemäß einer weiteren Ausführungsform wird das erfindungsgemäße Verfahren so durchgeführt, dass in Schritt A) als erste Adsorbenskomponente eine Verbindung der Formel R¹-E-L-X, R¹-L-E-X oder R¹-E-X bereitgestellt wird. Hierin stellt R¹ die lipophile Gruppe, E die hydrophile Gruppe, L einen Linker und X eine reaktive funktionelle Gruppe dar. Diese reaktive funktionelle Gruppe X wird so gewählt, dass sie in Schritt B) und/oder Schritt C) eine chemische Reaktion mit dem Fluid eingeht oder Wasserstoffbrücken mit diesem ausbilden kann. Aufgrund der Tatsache, dass das kontaminierte Fluid vorgegebenen ist (häufig eine wässrige Flüssigkeit), ist dem Fachmann bereits aufgrund einfachster organisch-chemischer Überlegungen klar, welche funktionellen Gruppen sich hierfür eignen. Der Linker L kann eine beliebige Gruppe sein, die nicht durch die Definition der hydrophilen Gruppe, der lipophilen Gruppe und der reaktiven Gruppe erfasst wird. Denkbar sind beispielsweise Alkylengruppen (beispielsweise CH₂ oder C₂H₄) zwischen E und X oder Ester- oder Ethergruppen zwischen R¹ und E.

Gemäß einer weiteren Ausführungsform kann die funktionelle Gruppe X, insbesondere im Fall eines wässrigen Lösungsmittels, eine, zwei oder mehrere Hydroxygruppen aufweisen. Hierdurch wird die Ausbildung von Wasserstoffbrückenbindungen zum Fluid unterstützt und zugleich auch die Hydrophilie der hydrophilen Gruppe erhöht. Eine Erhöhung der Hydrophilie kann im Einzelfall vorteilhaft sein.

Ferner kann die funktionelle Gruppe X so gewählt sein, dass insbesondere im Fall eines wässrigen Lösungsmittels, durch Reaktion mit dem Fluid eine, zwei oder mehrere Hydroxygruppen ausgebildet werden. Die Dauer dieser Reaktion kann aber - je nach Gruppe und Milieubedingungen - die Dauer des erfindungsgemäßen Verfahrens bei weitem übersteigen, so dass hier allenfalls von einer teilweisen Umsetzung auszugehen ist. Gemäß einer weiteren Ausführungsform kann daher die funktionelle Gruppe X ausgewählt sein aus Epoxid-Gruppen und Gruppen, die Schritt B) und/oder Schritt C) in einer S_{N}-Reaktion zu einer Hydroxygruppe reagieren. Als Gruppen, die in einer S_{N}-Reaktion zu einer Hydroxygruppe reagieren, sind insbesondere 2-Chlorethanol-Gruppen, Alkyl-Sulfonat-Gruppen, Brom-Alkyl-Gruppen oder Iod-Alkylgruppen zu nennen.

Gemäß einer weiteren Ausführungsform wird die erste Adsorbenskomponente so gewählt, dass in den Formel R¹-E-L-X, R¹-L-E-X oder R¹-E-X zwei oder mehr Gruppen X oder X-L an die hydrophile Gruppe gebunden sind. Es ergeben sich demnach insbesondere die Formeln R¹-E-(L-Xₙ), R¹-E-(L-X)ₙ, R¹-L-E-Xₙ oder R¹-E-Xₙ. Hierbei kann dann n zwei oder drei sein, im Fall der zuerst genannten Verbindung aber auch größer als 3 sein; L und X können jeweils gleich oder auch verschieden sein.

Liegen mehrere derartige Gruppen X vor, so kann X jeweils eine über einen Methylen-, Ethylen- oder Propylen-Linker gebundene Ether-Gruppe, insbesondere Ethyl- oder Methyl-Ether Gruppe sein. Mit derartigen multiplen Substuenten kann der gleiche Effekt wie bei einem einzigen Polyol-Substituent erzielt werden.

Ferner können als Gruppen X, zumindest zwei Alkohol-Gruppen oder Precursor, aus denen durch Reaktion mit dem Fluid Alkoholgruppen gebildet werden können, vorliegen. Insbesondere sind hierbei vicinale Diolgruppen beziehungsweise Precursorstrukturen hierfür zu nennen.

Gemäß einer weiteren Ausführungsform wird die funktionelle Gruppe X der ersten Adsorbenskomponente so gewählt, dass mit ihr zeit- und prozessabhängige Eigenschaften eingestellt werden können, insbesondere in Form eines pH-Regulators. So werden beispielsweise bei S_{N}-Reaktionen der Gruppe X mit Wasser im Fall von 2-Chlorethanol-Gruppen, Alkyl-Sulfonat-Gruppen, Brom-Alkyl-Gruppen und lod-Alkylgruppen Mineralsäuren freigesetzt. Diese Freisetzung erfolgt in situ, so dass beispielsweise im Fall von Aminen als hydrophiler Gruppe das für die Quaternisierung des Stickstoffs und die bessere Adsorption der fluorierten organischer Verbindung erforderliche "Quaternisierungsreagenz" H⁺ vor Ort erzeugt wird. Ferner können durch eine pH-Wert-Veränderung auch die Milieubedingungen eines zu reinigende wässrigen Gemischs verändert werden, sodass die Wechselwirkung des Addukts von erster Adsorbenskomponente und fluorierter organischer Verbindungen einerseits und zweiter Adsorbenskomponente andererseits verstärkt werden.

Die Ausführungen der vorstehenden sieben Absätze beziehen sich nicht ausschließlich für den Fall, dass als erste Adsorbenskomponente Verbindungen der Formel R¹-E-L-X, R¹-L-E-X oder R¹-E-X bereitgestellt werden. Sie gelten im Grundsatz auch, wenn Verbindungen der Formel X-L-R¹-E oder X-R¹-E vorliegen. Es hat sich allerdings als vorteilhaft erwiesen, wenn die reaktive Gruppe X, mittels der unter anderem die Hydrophilie erhöht werden kann, nicht an die lipophile sondern an die hydrophile Gruppe gebunden ist.

Gemäß einer weiteren Ausführungsform wird die erste Adsorbenskomponente so gewählt, dass sie eine besonders geringe aquatische Toxizität besitzt. Die grundsätzlich gut geeigneten quartären nicht polymeren Alkylamine (beispielsweise Hexadecyltrimethylammoniumchlorid, Dioctyldimethylammoniumchlorid oder quartäres Kokosalkylmethylaminthoxylatmethylchlorid) und Chloralkylamine (beispielsweise 3-chloro-2-hydroxypropyl-lauryl-dimethylammoniumchloride) weisen oft eine hohe aquatische Toxizität auf und sind gemäß CLP-Verordnung 1272/2008 sehr giftig für Wasserorganismen. Aminopropylester und Aminopropylamide (beispielsweise Undecylamidopropyltrimethylammoniummethosulfat, Docosylamidopropyldimethylamin oder 2-hydroxy-3-trimethylammoniumpropyldocosanoat), Aminodipropionate (beispielsweise 3-[2-Carboxyethyl(octyl)amino]propansäure oder Natriumoctyliminodiproponiat), quartären polymeren Alkylamine (beispielsweise das diquarternäres Polydimethylsiloxan Rewoquat SQ1 der Firma Evonik) weisen bereits eine deutlich niedrigere Toxizität auf. Im Hinblick auf die Toxizität am günstigsten sind aber Verbindungsklassen, die beispielsweise auch in Haarshampoos oder Weichspülern eingesetzt werden. Zu nennen sind hier Betaine (beispielsweise das TEGO Betain F50 der Firma Evonik) und insbesondere Triethanol Esterquats (beispielsweise Dioleoylethylhydroxyethylmonium Methosulfat oder entsprechende Verbindungen, bei denen die Oleyl-Einheiten zumindest teilweise durch gesättigte C15 bis C18-Alkylgruppen ersetzt sind).

Gemäß einer weiteren Ausführungsform wird in Schritt A) eine feste Adsorbenskomponente bereitgestellt, die eine nichtionische Oberfläche aufweist. Hierunter wird eine Oberfläche verstanden, auf der zwar temporär Ladungen vorliegen können, etwa weil es sich um einen elektrischen Leiter mit Graphit-Strukturelementen handelt, bei der aber keine dauerhaften ionischen Zentren auf der Oberfläche vorliegen. Zu nennen ist beispielsweise Aktivkohle, deren Oberfläche zwar partiell negativ geladen sein kann, bei der es sich bekanntlich aber nicht um eine Verbindung mit ionischer Struktur handelt.

Die feste Adsorbenskomponente kann insbesondere ein modifiziertes oder unmodifiziertes Adsorbens sein, ausgewählt aus Aktivkohlen, Aluminiumoxiden, Kieselgelen, Rußen, Zeolithen, Silica Gelen, Tonen und faserförmigen oder mikrokristallinen Cellulosen (etwa Perlcellulosen). Um eine bessere Adsorption des Addukts aus fluorierter organischer Verbindung und erster Adsorbenskomponente zu erreichen, sollte die Oberfläche der festen Adsorbenskomponente möglichst lipophil sein. Hierfür können die vorstehend genannten festen Adsorbenskomponenten lipophil modifiziert sein; als Beispiel hierfür seien lipophil modifizierte Aluminiumoxide und hydrophob modifizierte Percellulosen genannt.

In Ihrer Eigenschaft als herkömmliches festes Adsorbens weist die zweite Adsorbenskomponente vorzugsweise eine möglichst große spezifische Oberfläche auf (die mittels DIN ISO 9277:2003-05 bestimmt werden kann). Im Regelfall wird die spezifische Oberfläche mindestens 10¹ m²/g sein und insbesondere 5*10² bis 5*10³ m²/g, beispielsweise 9*10² bis 1,5*10³ m²/g betragen.

Gemäß einer weiteren Ausführungsform besitzt die zweite Adsorbenskomponente einen mittleren Partikeldurchmesser von 0,005 bis 6 mm, insbesondere 0,02 bis 4 mm beispielsweise 0,05 bis 1 mm. Die Partikelgröße wird hierbei mittels Siebverfahren bestimmt. Die Partikelform kann beliebig sein, beispielsweise sphärisch oder auch nadelförmig. Auch die Form der Porosität des Adsorbens ist beliebig wählbar von makrobis mikroporös.

Gemäß einer weiteren Ausführungsform wird das erfindungsgemäße Verfahren so durchgeführt, dass vor Schritt D) (und üblicherweise auch nach Schritt C) sowie nach Schritt B)) ein Flockungshilfsmittel zugesetzt wird (im Einzelfall kann der Zusatz aber auch gleichzeitig mit oder vor Schritt B) und/oder C erfolgen, etwa weil die Flockung erst nach Adsorption des Kontaminats erfolgt). Grundsätzlich ist ein derartiges Verfahren bei sehr kleinen Partikelgrößen der festen Adsorbenskomponente sinnvoll und den mit diesen Partikelgrößen einhergehenden größeren Oberflächen von Vorteil (somit sind die im vorstehenden Absatz genannten Bereiche insbesondere hinsichtlich der oberen Grenze gültig). Insbesondere geeignet für diese Ausführungsform sind Adsorbentien mit mittleren Partikelgrößen von 0,005 mm bis zu maximal 1 mm, beispielsweise bis zu maximal 0,5 mm Partikeldurchmesser. Im Regelfall wird die feste Adsorbenskomponente in feinverteilter Form in das kontaminierte Fluid gegeben, so dass eine Adsorption erfolgen kann. Anschließend wird das beladene Adsorbens-Kit mit einem Flockungshilfsmittel zur Flockung gebracht. Es entstehen auf diese Weise mit Schadstoff beladene, größere Flocken, die leicht sedimentieren bzw. flotieren und/oder durch Filtration abgetrennt werden können. Bei Auswahl geeigneter Flockungshilfsmittel kann die Menge der abgetrennten Organofluorverbindungen zusätzlich gesteigert werden. Als Flockungshilfsmittel sind beispielsweise kationische, insbesondere aber anionische Polyelektrolyte geeignet. Diese können zum Beispiel auf Polyacrylat, Polyacrylamid, Polyethylenimin und Polyethylenoxid basieren. Daneben können auch nichtionische synthetische und natürliche Flockungshilfsmittel (beispielsweise Stärke und Leim) oder anorganische Polymere wie Polyaluminiumverbindungen, auch in Kombination mit mehrwertigen, niedermolekularen Aluminium- oder Eisensalzen eingesetzt werden.

Die Verwendung des erfindungsgemäßen Adsorbens-Kits in Kombination mit einem Flockungshilfsmittel hat den Vorteil, dass sich die die Organofluorverbindungen enthaltenden Flockungsprodukte (beispielsweise PFT-haltige Flockungsprodukte) meist besonders stabil in ihrem Aufbau zeigen; der Schadstoff ist dann in der Flocke dauerhaft immobilisiert und neigt nicht zum Auswaschen.

Gemäß einer weiteren Ausführungsform wird das erfindungsgemäße Verfahren so durchgeführt, dass zusätzlich zu der ersten Adsorbenskomponente ein nichtionisches Tensid eingesetzt wird und zwar insbesondere zusammen mit der ersten Adsorbenskomponente und insbesondere in Schritt B). Mittels eines derartigen nichtionischen Tensids kann bei bestimmten Kombinationen aus erster Adsorbenskomponente und Organofluorverbindung eine Stabilisierung des gebildeten Addukts und/oder eine Stabilisierung von gebildeten Mizellen erfolgen. Im Ergebnis kann durch ein derartiges Cotensid also eine Effizienzsteigerung erzielt werden. Zu nennen sind als nichtionische Tenside (Oligo)oxyalkylen-Verbindungen (beispielsweise Fettalkoholpolyglycolether), Kohlenhydrat-Verbindungen (beispielsweise Alkylpolyglucoside, Saccharoseester, Sorbitanester und Fettsäure-*N*-methylglucamide) und Aminoxide (beispielsweise Alkyldimethylaminoxide).

Gemäß einer weiteren Ausführungsform wird das erfindungsgemäße Verfahren in einem Rührreaktor durchgeführt oder unter Bedingungen, die denen in einem Rührreaktor entsprechen. Das erfindungsgemäße Verfahren kann unter diesen Bedingungen seine Vorteile besonders gut zur Geltung bringen. Es ist erfindungsgemäß nämlich nicht unbedingt nötig, dass das kontaminierte Fluid über eine Kolonne mit den Adsorbentien geleitet wird. Vielmehr ist es ausreichend, wenn die beiden Adsorbenskomponenten mit dem Fluid in irgendeiner Form in Kontakt gebracht wird und dann eine Durchmischung von Adsorbenskomponenten und Fluid erfolgt. Durch die starken Wechselwirkungen zwischen fluorierter organischer Verbindung und Adsorbenskomponenten ist eine derartige Durchmischung, etwa in einem Rührreaktor, völlig ausreichend um eine gute Abtrennung der fluorierten organischen Verbindung zu gewährleisten. Um eine besonders schnelle Abtrennung zu ermöglichen kann es aber sinnvoll sein, ein Flockungshilfsmittel zuzusetzen (das bei Verwendung in einer Kolonne oder Trennsäule nicht erforderlich wäre). Grundsätzlich kann das erfindungsgemäße Verfahren aber nicht nur in einem Rührreaktor (bei dem die feste Adsorbenskomponente häufig pulverförmig eingesetzt wird) sondern auch in einer Kolonne oder einem Festbettadsorber (bei dem die feste Adsorbenskomponente häufig in granulärer Form eingesetzt wird) durchgeführt werden.

Das erfindungsgemäße Verfahren wird - ohne die Offenbarung auf diese Ausführungen beschränken zu wollen - nachfolgend noch näher beschrieben:
Nach Bereitstellung der Komponenten gemäß Schritt A) wird zunächst dem kontaminierten Fluid, aus dem fluorierte organische Verbindungen entfernt werden sollen, ein amphiphiles, kationisches Additiv (als erste Adsorbenskomponente) unter Rühren (∼ 100 rpm) zugegeben. Zur Einstellung einer geeigneten Hydrophob-Hydrophil-Balance wird eine Verbindung gewählt, die mit Amino-Gruppen und/oder quaternären AmmoniumGruppen versehen ist und ferner für den liphohilen Anteil mit längeren Alkylketten (z.B. Cocoalkyl, Stearyl oder auch Octyl oder Dodecyl) ausgestattet ist. Funktionalitäten wie Aryl- und Benzyl-Gruppen ermöglichen ebenfalls die erwünschte Wechselwirkung und Bildung von Addukten mit fluorierten organischen Verbindungen. Die genannten Aryl-, Aralkyl- und Alkyl-Gruppen können dabei auch teilfluoriert sein. In typischer Weise wird die Gesamtkonzentration der im Fluid vorliegenden fluorierten organischen Verbindungen zwischen 0,001 und 100 mg/L liegen; die Konzentration der zugesetzten ersten Adsorbenskomponente wird dann üblicherweise zwischen 0,1 und 1000 mg/L, häufig 1 bis 100 mg/L betragen. Ab Konzentrationen 0,2 mmol/L, häufig 1 bis 10 mmol/L ist abhängig von der ersten Adsorbenskomponente die vorteilhafte Bildung von Micellen zu erwarten. Häufig wird die Konzentration ersten Adsorbenskomponente gleich hoch oder höher als die der zu erwartenden Konzentration der abzutrennenden fluorierten organischen Verbindung sein, beispielsweise zumindest 50 mal bis 1000 mal so hoch (bezogen auf die Gewichtsanteile). Aus der Wechselwirkung der beiden Komponenten geht zumindest zu wesentlichen Teilen ein Addukt hervor, wobei die Stöchometrie des aus den Komponenten gebildeten Adduktes von der Konzentration und dem chemischen Aufbau der Reaktanden abhängig ist. Die Kontaktzeit kann einen Zeitraum von Sekunden bis Stunden umfassen, häufig liegt diese im Bereich von 1 bis 30 Minuten.

Im Schritt B) wird das Fluid mit der zweiten Adsorbenskomponente in Kontakt gebracht, so dass das gebildete Addukt, als auch Anteile an noch freiem Schadstoff und meist auch erste Adsorbenskomponente auf der zweiten Adsorbenskomponente gebunden werden. In typischer Weise findet dieser Prozess in einem Rührreaktor statt, indem dem im Fluid (das bereits mit der ersten Adsorbenskomponente versehen wurde) die zweite Adsorbenskomponente, z.B. Pulverkohle unter Rühren (ca. 100 rpm) dispergiert wird. Die Konzentration der eingebrachten festen Adsorbenskomponente liegt zwischen 1 und 10000 mg/L, bevorzugt zwischen 10 und 1000 mg/L, die Rührzeit kann wiederum einen Zeitraum von Sekunden bis Stunden umfassen, häufig liegt diese im Bereich von 1 bis 30 Minuten. Alternativ kann das Adsorbens in einer Adsorptionssäule vorliegen und mit dem kontaminierten Fluid durchströmt werden, um die erwünschte Adsorption zu erzielen.

Der nächste Schritt beinhaltet die Abtrennung des gereinigten Fluides von dem beladenen Adsorbens mit Hilfe eines Flockungshilfsmittels. Dieser Prozess der Fest-Flüssig-Trennung erfolgt im Rührreaktor; es wird typischerweise Flockungshilfsmittel in einer Konzentration von 0.1 - 10 mg/L zugesetzt. Dabei wird mehrere Minuten gerührt (ca. 100 rpm).

Anschließend an diesen Schritt erfolgt (auch wenn kein Flockungshilfsmittel zugesetzt wird) die Sedimentation ohne Rühren. Das Sediment wird über eine Filtervorrichtung abgetrennt. Falls nötig, kann das so gereinigte Fluid einer weiteren Behandlung mit einem Adsorbens unterzogen werden oder auch die erfindungsgemäße Behandlung nochmals durchlaufen.

Die erfindungsgemäße Aufgabe wird ferner durch ein Kit zur Abtrennung fluorierter organischer Verbindungen aus kontaminierten, in Form wässriger Flüssigkeiten vorliegender Fluide gelöst. Dieses Kit umfasst eine erste Adsorbenskomponente, die flüssig ist oder in eine flüssige Form bringbar ist und zumindest teilweise in gelöster Form vorliegt und eine zweite (feste) Adsorbenskomponente. Die erste Adsorbenskomponente ist eine chemische Verbindung, die eine lipophile Gruppe und eine hydrophile Gruppe enthält, oder eine derartige Verbindung in gelöster Form enthält, wobei die hydrophile Gruppe zumindest eine kationische Gruppe enthält, die ein Amin, ein organisch substituiertes Ammonium, ein organisch substituiertes Phosphonium oder einen Metallkomplex umfasst, und wobei die lipophile Gruppe ausgewählt ist aus Alkylgruppen, die zumindest eine Octyleneinheit umfassen, aus Aryl-Gruppen und aus Aralkyl-Gruppen. In flüssige Form bringbar heißt hierbei auch, dass diese flüssige Form erst durch Lösung im zu reinigenden Fluid erfolgt oder durch Auflösen in einem beliebigen anderen Lösungsmittel, das mit dem Fluid mischbar ist.

Weitere vorteilhafte Ausgestaltungen des Kits sind vorstehend bei der Beschreibung des erfindungsgemäßen Verfahrens erläutert.

Die erfindungsgemäße Aufgabe wird schließlich auch durch Verwendung einer Verbindung der Formeln R¹-E-L-X, R¹-L-E-X oder der Formel R¹-E-X zur Abtrennung fluorierter organischer Verbindungen aus kontaminierten, in Form wässriger Flüssigkeiten vorliegender Fluide gelöst. Hierbei ist R¹, E, L und X wie vorstehend definiert. Auch hierzu sind weitere vorteilhafte Ausgestaltungen vorstehend bei der Beschreibung des erfindungsgemäßen Verfahrens erläutert.

Das erfindungsgemäße Verfahren, das hierfür verwendete Kit und auch die als erste Adsorbenskomponente eingesetzten Verbindungen der Formeln R¹-E-L-X, R¹-L-E-X oder der Formel R¹-E-X sind insbesondere geeignet zur Abtrennung fluorierter organischer Verbindungen. Hierbei sind insbesondere fluorierte Kohlenwasserstoffe zu nennen, wie perfluorierte Verbindungen, teilfluorierte Tenside oder perfluorierte Tenside. Grundsätzlich ist hierbei auch möglich, dass eine Desorption der adsorbierten Verbindungen (Addukt) und damit eine Regenerierung der Trennphase (Aktivkohle) sowie die Wiedergewinnung der fluorierten Verbindungen möglich ist, insbesondere wenn es sich hierbei um Wertstoffe handelt, die ansonsten mit aufwändigen chemischen Reaktionen erst wieder hergestellt werden müssten. Eine derartige Desorption kann beispielsweise erfolgen, wenn schwach basische Aminogruppen in der ersten Adsorbenskomponente vorliegen und mit Anionen von beispielsweise perfluorierten Tensiden Addukte bilden. Die Säureanionen der Tenside sind dabei reversibel gebunden und mit einem Zusatz an Base werden bei der Regeneration die freien Aminogruppen wieder zurückgebildet. Abhängig vom Typ der Aminogruppe (primäre, sekundäre oder tertiäre Aminogruppe) und von der Konstitution des Adduktes ist der pH-Wert der optimalen Regeneration abhängig bzw. einstellbar.

Zusammenfassend ist festzustellen, dass das neue Verfahren speziell an den Stand der Technik und die moderne Praxis der Wasseraufbereitung von PFT-kontaminierten Wässern anknüpft und diese durch die Verwendung von spezifischen Carrierverbindungen (erfindungsgemäß: ersten Adsorbenskomponenten) verbessert. Dadurch lassen sich kommerzielle Adsorbentien und ihre etablierten Wege der Regeneration wie die thermische Reaktivierung von Aktivkohlen weiterhin nutzen und zugleich über einen verringerten Materialeinsatz sowohl Anlagen- als auch Prozesskosten einsparen.

Weitere vorteilhafte Ausführungsbeispiele und Weiterbildungen der Erfindung ergeben sich im Folgenden - ohne Einschränkung der Allgemeinheit - aus den Beispielen.

### Adsorption im Batchverfahren:

Nachfolgend wird die Adsorption im Batchverfahren erläutert und die Leistungsfähigkeit der Adsorptionsmethode unter Verwendung verschiedener Reagenzien gezeigt. Hierbei erfolgt der Einsatz in einer vier- bzw. sechs-stufigen RRS-Anlage (Rührreaktor-Sorptions-Anlage).

Die Verfahrensstufen umfassen:
Stufe 1: Dosierung / Reagenz 1: erste Adsorbenskomponente
Stufe 2: Dosierung / Reagenz 2: zweite Adsorbenskomponente
Stufe 3: Dosierung / Reagenz 3: Flockungshilfsmittel
Stufe 4: Sedimentation
Stufe 5: Dosierung / Reagenz 4: zweite Adsorbenskomponente
Stufe 6: Sedimentation

Je nach Beispiel variieren die eingesetzten Reagenzien und die Anzahl der Stufen.

Jeweils 800 ml PFT-haltiges Rohwasser werden mit einem Messzylinder abgemessen und in einem 1000 ml Becherglas vorgelegt. Insgesamt werden vier Proben vorbereitet. Als PFT-haltiges Rohwasser wird hierbei eine wässriges Gemisch, das folgende PFT enthält, eingesetzt: Perfluoroctansulfonat (PFOS) 2,98 µg/l, Perfluorheptansulfonat (PFHpS) 0,27 µg/l, Perfluorhexansulfonat (PFHxS) 3,90 µg/l, Perfluorbutansulfonat (PFBS) 0,54 µg/l, Perfluoroctansäure (PFOA) 0,36 µg/l, Perfluorheptansäure (PFHpA) 0,13 µg/l, Perfluorhexansäure (PFHxA) 0,64 µg/l, Perfluorpentansäure (PFPeA) 0,21 µg/l, Perfluorbutansäure (PFBA) 0,23 µg/l.

Die abgedeckten Bechergläser werden anschließend in dem Reihenrührwerk platziert. Sobald die vorgesehene Menge an erster Adsorbenskomponente (Reagenz 1) dem jeweiligen Becherglas zugeführt wurde, wird das Rührblatt in das Medium eingetaucht, das Rührwerk eingeschaltet und die Drehzahl auf den vorgesehenen Wert eingestellt.

Die Dosierung der zweiten Adsorbenskomponente (Reagenz 2) und des Flockungshilfsmittels (Reagenz 3) erfolgt jeweils nach Ablauf der vorgesehenen Reaktionszeiten. Die Rührerdrehzahl wird dabei für die einzelnen Versuchsphasen spezifisch angepasst. Nach Ablauf der dritten Stufe beginnt die Sedimentationsphase. Hierzu wird das Rührwerk ausgeschaltet und die Rührblätter aus den Proben entfernt.

Nach Ablauf der Sedimentationszeit werden die in den Proben enthaltenen Feststoffe durch Filtration abgetrennt. Das Filtrat wird in 1000 ml Bechergläsern aufgefangen und für dessen weitere Analyse (PFT-Konzentration) werden möglichst kleine Probenvolumen entnommen.

Die Rührblätter und Rührwellen des Reihenrührwerks werden nochmals gereinigt, sofern nachfolgend ein weiterer Adsorptionsschritt (Reagenz 4) mit dem Filtrat durchgeführt wird. Nach Ablauf der zweiten Sedimentationszeit wird das in der Probelösung enthaltene Sediment mittels Filtration abgetrennt und das verbleibende Filtrat wiederum analysiert.

### Beispiel 1

a) Eingesetzte Reagenzien:
Reagenz 1: Herstellung von 50 ml Reagenzlösung 49,5 g destilliertes Wasser und 0,5 g Quab 342 (40%ige wässrige Lösung von 3-Chloro-2-hydroxy-propyldimethyldocecyl-ammoniumchlorid; Firma Quab Chemicals Inc., USA)
Reagenz 2: Pulveraktivkohle auf Basis von Steinkohle (der Firma Cornelsen Umwelttechnologie, Typ: PFT Sorb, BET 900 m²/g)
Reagenz 3: Flockungshilfsmittel (der Firma Ashland, Typ: Praestol A4030L) Herstellung einer 0,1%igen-Flockungshilfsmittel-Dosierlösung aus 99,9 g destilliertem Wasser und 0,1 g Flockungshilfsmittel
Reagenz 4: wie Reagenz 2

b) Verfahrensschritte und Einstellungen:
Phase 1: Dosierung von Reagenz 1 jeweils 1 ml/l; Drehzahl: 200 min⁻¹;
Reaktionszeit: 30 Minuten
Phase 2: Dosierung von Reagenz 2; Drehzahl: 200 min⁻¹; Reaktionszeit: 30 Minuten

| Ansatz-Nr. | 1 | 2 | 3 | 4 |
|---|---|---|---|---|
| Reagenz 2 [mg/l] | 25 | 50 | 75 | 100 |

Phase 3: Dosierung von Reagenz 3 jeweils 1 ml/l; Drehzahl: zunächst 200 min⁻¹ nach 1 Minute 80 min⁻¹; Reaktionszeit: 15 Minuten
Phase 4: Sedimentation; Sedimentationszeit: 5 Minuten
Phase 5: Filtration; Filtertyp: Faltenfilter Whatman 597 ½
Phase 6: Dosierung von Reagenz 4 Dosierung von Reagenz 1 jeweils 25 ml/l; Drehzahl: 200 min⁻¹; Reaktionszeit: 30 Minuten
Phase 7: Filtration; Filtertyp: Faltenfilter Whatman 597 ½

### Beispiel 2

Die Durchführung erfolgt wie in Beispiel 1, allerdings wird als Reagenz 1 hydrolysiertes 3-Chloro-2-hydroxy-propyldimethyldocecylammoniumchlorid (Hydrolysegrad ca. 12 mol%) eingesetzt.

### Beispiel 3

Die Durchführung erfolgt wie in Beispiel 1, allerdings wird als Reagenz 1 (1-Dodecyl)trimethylammoniumchlorid (Firma Alfa Aesar) eingesetzt.

### Beispiel 4

Die Durchführung erfolgt wie in Beispiel 1, allerdings wird als Reagenz 1 (1-Tetradecyl)trimethylammoniumchlorid (Firma Alfa Aesar) eingesetzt.

### Beispiel 5 Versuch ohne erste Adsorbenskomponente

Die Durchführung erfolgt wie in Beispiel 1; statt Reagenz 1 wird die gleiche Menge destilliertes Wasser zudosiert.

### Vergleich von Beispiel 1 bis 4 und Beispiel 5

Aus dem Vergleich zeigt sich deutlich wie vorteilhaft das erfindungsgemäße Verfahren ist. In Tabelle 1 ist die nach Durchführung der Versuche 1 bis 5 Im Filtrat verbliebene Restkonzentration an PFT in µg/L angegeben (unter Konzentration [Konz.] PFTsorb = 0 sind Blindversuche angegeben):

**Tabelle 1:**

| | Konzentration PFT [µg/L] | | | | |
|---|---|---|---|---|---|
| Konz. PFTsorb [mg/L] | Beispiel 5 | Beispiel 1 | Beispiel 2 | Beispiel 3 | Beispiel 4 |
| 0 | 8,89 | 10,65 | 9,95 | 9,95 | 9,95 |
| 25 | 4,69 | 2,08 | 3,83 | 3,65 | 3,23 |
| 50 | 4,84 | 1,08 | 2,08 | 1,09 | 1,1 |
| 75 | 3,73 | 0,74 | 1,03 | 0,62 | 0,65 |
| 100 | 2,75 | 0,48 | 0,54 | 0,34 | 0,32 |
| 125 | 2,5 | | | | |
| 150 | 1,94 | | | | |
| 175 | 1,09 | | | | |
| 200 | 0,78 | | | | |

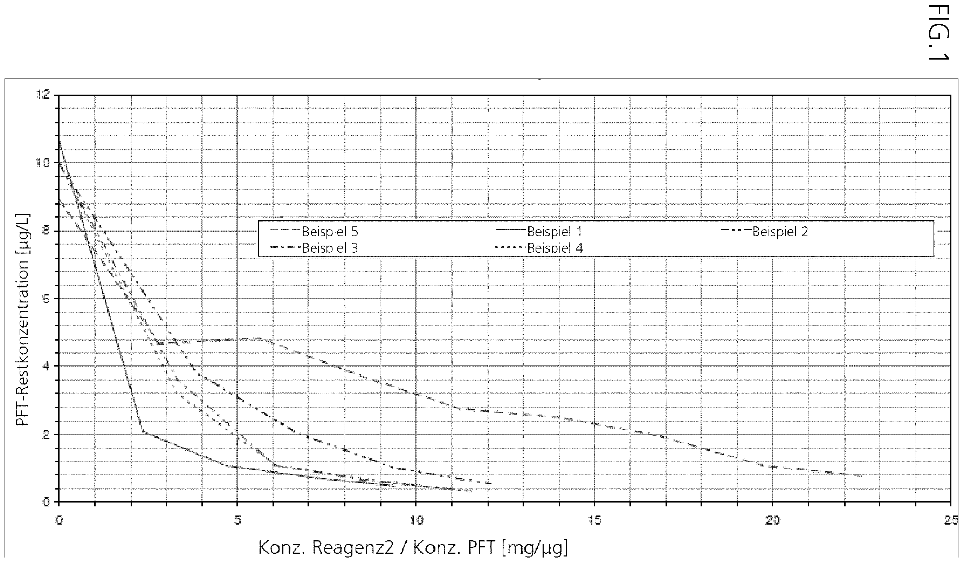

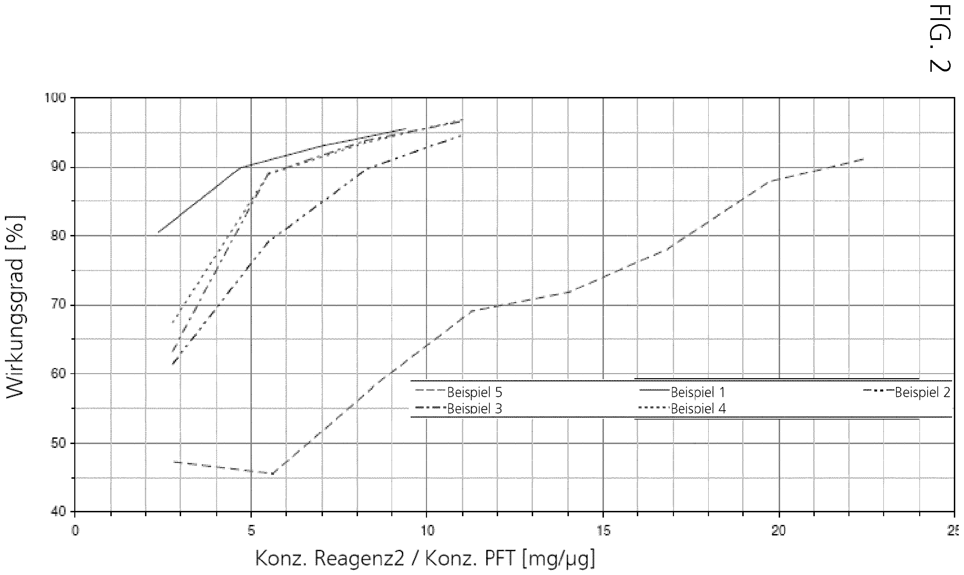

Figur 1 zeigt die graphische Aufbereitung der Ergebnisse der Versuchsreihen aus Tabelle 1. Die Aktivkohle ohne Zusatz der ersten Adsorbenskomponente (gestrichelte Linie ---) zeigt die schlechtesten Ergebnisse. Beispiel 1 (durchgezogene Linie) die besten. Durch Zusatz von Chloro-2-hydroxy-propyldimethyldocecylammoniumchlorid können also deutlich geringe Mengen an Adsorbens eingesetzt werden. Zwischen diesen Ergebnissen liegen Beispiel 2 (Strichpunkt-Linie -..-), Beispiel 3 (Strichpunkt-Linie -.-.) und Beispiel 4 (gepunktete Linie). Figur 2 verdeutlicht diese Aussage nochmals durch Auftragung des erzielten Wirkungsgrades in Abhängigkeit vom Verhältnis von Adsorbens und PFT-Adsorptiv.

### Beispiel 6 bis 10Versuchsreihen ohne erste Adsorbenskomponente

Die Durchführung erfolgt wie in Beispiel 5. Statt Pulveraktivkohle wird eine andere zweite Adsorbenskomponente einer Menge von 100 mg/L verwendet. Dies ist Knochenmehl (pulverförmig gemahlene Rinderknochen mit einem Rohaschegehalt von 81,4% - Beispiel 6), Titandioxid (pulverförmig - Beispiel 7), granuläre Stärke "Amylofax 75" der Firma AVEBE (Beispiel 8), granuläre Stärke "Wisprofloc P" der Firma AVEBE (Beispiel 9) und granuläre Stärke "Avecat 15" der Firma AVEBE (Beispiel 10). Im Vergleich zur Aktivkohle besitzen diese eine hydrophilere Oberflächenchemie - hierdurch resultieren kleinere Wirkungsgrade für die PFT-Adsorption.

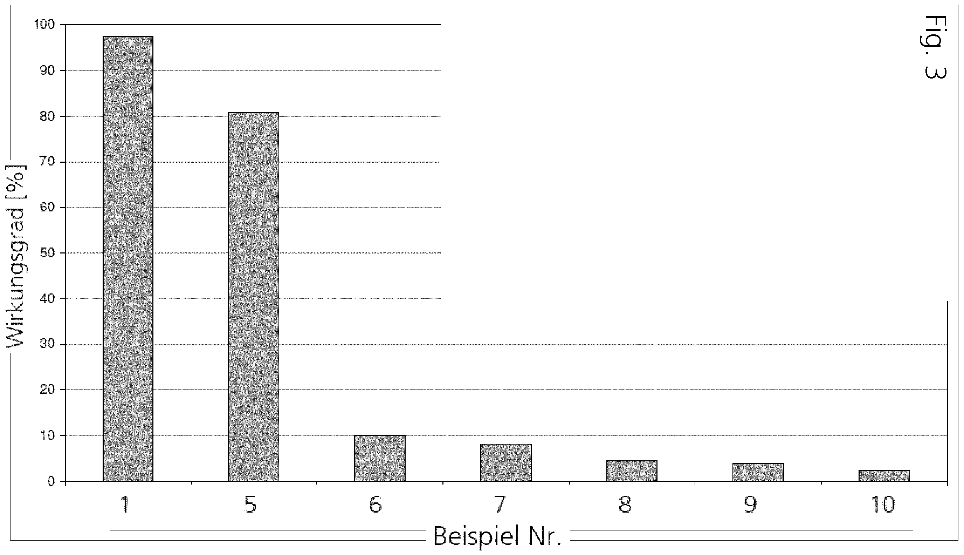

Figur 3 zeigt die erzielten Wirkungsgrade aus den Beispielen 1 und 5 bis 10 in Abhängigkeit vom der festen Adsorbenskomponente

### Beispiel 11

Die Durchführung erfolgt wie in Beispiel 1, allerdings wird Rohwasser mit den Inhaltsstoffen gemäß den Angaben der nachfolgenden Tabelle 2 eingesetzt. Ferner entfallen die Stufen 3, 4 und 5 und bei den Vergleichsversuchen Aktivkohle 1 und 2 auch die Stufe 1. Schließlich wurde die Adsorbenskomponente 1 in einer Menge von 22,5 mg/l und die Adsorbenskomponente 2 in einer Menge von 100 mg/l eingesetzt.

Tabelle 2 zeigt jeweils zwei zusammengehörige Spaltenpaare, bei denen die linke Spalte die Konzentration des jeweiligen PFT im Rohwasser und die rechte Spalte die durch die Adsorbenskomponenten abgetrennen Mengen dieser PFTs angibt. Man erkennt, dass durch das erfindungsgemäße Kit ein deutlich besserer Wirkungsgrad als mit reiner Aktivkohle erreicht werden kann.

**Tabelle 2 (alle Werte besitzen die Einheit µg/l)**

| | Rohwasser | Aktivkohle 1 | Rohwasser | Aktivkohle 2 | Rohwasser | Quab 342 + Aktivkohle 2 |
|---|---|---|---|---|---|---|
| PFBA | 0,56 | 0,09 | 0,46 | 0,18 | 0,65 | 0,52 |
| PFPeA | 1,6 | 0,81 | 1,4 | 1,01 | 1,9 | 1,86 |
| PFHxA | 2,2 | 1,88 | 2,1 | 2,03 | 3 | 3 |
| PFHpA | 0,4 | 0,39 | 0,43 | 0,43 | 0,48 | 0,48 |
| PFOA | 0,87 | 0,87 | 0,8 | 0,8 | 0,92 | 0,92 |
| PFBS | 1,00 | 0,90 | 0,96 | 0,96 | 1,1 | 1,1 |
| PFHxS | 6,40 | 6,35 | 4 | 4 | 7,6 | 7,6 |
| PFOS | 11 | 10,9 | 5,9 | 5,9 | 11 | 11 |
| Summe PFTs | 24,03 | 22,19 | 16,05 | 15,31 | 26,65 | 26,48 |
| Wirkungsgrad | | 92,3 % | | 95,4 % | | 99,4 % |

| | | | | | | |
|---|---|---|---|---|---|---|
| Aktivkohle 1 = Donau Carbon, Hydraffin P1000 Aktivkohle 2 = Pulveraktivkohle auf Basis von Steinkohle (der Firma Cornelsen Umwelttechnologie, Typ: PFT Sorb, BET 1000 m²/g) | | | | | | |

### Beispiel 12

Die Durchführung erfolgt wie in Beispiel 11 (auch hinsichtlich der eingesetzten Mengen der Adsorbenskomponenten), allerdings wird Rohwasser mit den Inhaltsstoffen gemäß den Angaben der nachfolgenden Tabelle 3 eingesetzt. Ferner entfallen die Stufen 3, 4 und 5, bei den Vergleichsversuchen Aktivkohle 1 bis 3 zusätzlich die Stufe 1 und bei dem Versuch "TEA-Esterquat" zusätzlich die Stufe 2.

Tabelle 3 zeigt in der Spalte ganz links die Konzentration des jeweiligen PFT im Rohwasser und in den Spalten rechts daneben die durch die Adsorbenskomponenten abgetrennen Mengen dieser PFTs. Man erkennt, dass bereits durch die erste Adsorbenskomponente ohne zusätzliche feste Adsorbenskomponente fast der Wirkungsgrad erzielt wird, der auch durch das erfindungsgemäße Kit erreicht wird. Bei Verwendung von TEA-Esterquat kann hier auf die Aktivkohle bereits deshalb verzichtet werden, weil nach Kontaktierung dieser Verbindung mit dem Rohwasser ein Ausfällen des gebildeten Addukts zu beobachten ist. Das eingesetzte TEA-Esterquat ist ein Ölsäurebasiertes Esterquat, das erhältlich ist durch dreifache Veresterung von Triethanolamin mit ungesättigten C18-Fettsäuren (auf Ölsäure-Basis) und nachfolgende Quaternisierung mit Dimethylsulfat. Die Löslichkeit beträgt 4 mg/L (bei 20°C und pH 7,1); der Schmelzpunkt beträgt 4 °C und die Dichte 1,059 g/cm³ (bei 20 °C) Das TEA-Esterquat wird als Lösung in 2-Propanol eingesetzt.

In jedem Fall weisen beide Beispiele einen deutlich besseren Wirkungsgrad auf als er mit reiner Aktivkohle erreicht werden kann. Daneben zeigt sich, dass dieser Effekt noch stärker zu Tage tritt, wenn man nur die PFTs mit 6 oder weniger Kohlenstoffatomen in der Fluoralkylgruppe betrachtet.

**Tabelle 3 (alle Werte besitzen die Einheit µg/l)**

| | Rohwasser | Aktivkohle 1 | Aktivkohle 2 | Aktivkohle 3 | TEA-Esterquat | TEA-Esterquat + Aktivkohle 2 |
|---|---|---|---|---|---|---|
| PFBA | 6,9 | 0,5 | 0,5 | 0,7 | 0,8 | 0,9 |
| PFPeA | 16 | 4 | 4 | 3 | 4 | 5 |
| PFHxA | 33 | 6 | 11 | 4 | 12 | 12 |
| PFHpA | 5,5 | 2,5 | 4,1 | 1,6 | 4,1 | 3,9 |
| PFOA | 12 | 6,8 | 10,4 | 4,1 | 10,5 | 10,5 |
| PFNoA | 0,13 | 0,09 | 0,13 | 0,06 | 0,12 | 0,12 |
| PFDeA | 0,09 | 0,09 | 0,09 | 0,07 | 0,09 | 0,09 |
| PFOSA | 0,62 | 0,56 | 0,62 | 0,33 | 0,58 | 0,59 |
| PFBS | 31 | 13 | 16 | 7 | 24,5 | 23,6 |
| PFHxS | 170 | 112 | 150 | 60 | 160,6 | 160,7 |
| PFHpS | 24 | 21,1 | 23,39 | 16,2 | 23,59 | 23,61 |
| PFOS | 500 | 447 | 487 | 350 | 475 | 486 |
| 4:2 FTS | 0,25 | 0,21 | 0,24 | 0,17 | 0,21 | 0,21 |
| 6:2 FTS | 25 | 19,5 | 23,7 | 10 | 20,7 | 20,4 |
| 8:2 FTS | 13 | 12,32 | 12,85 | 9,6 | 12,49 | 12,57 |
| Summe PFTs | 837,49 | 645,67 | 744,01 | 466,83 | 749,28 | 760,19 |
| Wirkungsgrad | | 77,1 % | 88,8 % | 55,7 % | 89,5 % | 90,8 % |
| Summe C4-C6 | 282,15 | 155,21 | 205,44 | 84,87 | 222,81 | 222,81 |
| Wirkungsgrad C4-C6 | | 55,0 % | 72,8 % | 30,1 % | 79,0 % | 79,0 % |

| | | | | | | |
|---|---|---|---|---|---|---|
| Aktivkohle 3 = Reagenz 2 PFNoA = Perfluornonansäure, PFDeA = Perfluordecansäure; PFOSA = Perfluoroctansäureamid, n:2 FTS = n:2 Fluorotelomersulfonsäure CₙF₂ₙ₊₁C₂H₄SO₃H | | | | | | |

### Beispiel 13

Es wurden weitere als erste Adsorbenskomponente geeignete Verbindungen untersucht, ohne dass zusätzlich eine feste Adsorbenskomponente eingesetzt wurde. Wie im Fall des TEA-Esterquat (Beispiel 12) kann auf den Zusatz Aktivkohle bereits deshalb verzichtet werden, weil nach Kontaktierung dieser Verbindungen mit dem Rohwasser ein Ausfällen des gebildeten Addukts zu beobachten ist.

Ersetzt man das TEA-Esterquat aus Beispiel 12 unter ansonsten identischen Versuchsbedingungen durch eine Lösung von 80% Dioleoylethyl-hydroxyethylmonium Methosulfat in 20% Glycol oder alternativ durch Tego Betain F 50 der Firma Evonik (das Alkylamidopropylbetain: 1-Propanaminium-3-amino-N-carboxymethyl-N,N-dimethyl-N-C₈-C₁₈ acyl-Derivat) so sind auch bei Variation der eingesetzten Rohwässer jeweils Fällungsprodukte zu beobachten, die in Abhängigkeit von Rohwasser und erster Adsorbenskomponente unterschiedlich in Art (Flockenstruktur) und Menge waren. In jedem Fall zeigten sich visuell gut wahrnehmbare Fällungsprodukte und eine deutlich verminderte PFT-Restkonzentration in dem behandelten Rohwasser.

## Patentansprüche

1. Verfahren zur Abtrennung fluorierter organischer Verbindungen aus kontaminierten wässrigen Flüssigkeiten mittels zumindest einer ersten Adsorbenskomponente mit folgenden Schritten
A) Bereitstellung der ersten Adsorbenskomponente, wobei die erste Adsorbenskomponente eine chemische Verbindung ist, die eine lipophile Gruppe und eine hydrophile Gruppe enthält, und vorzugsweise eine derartige Verbindung in gelöster Form enthält,
wobei die hydrophile Gruppe zumindest eine kationische Gruppe enthält,
wobei die lipophile Gruppe ausgewählt ist aus Alkylgruppen, die zumindest eine Octyleneinheit umfassen, aus Aryl-Gruppen und aus Aralkyl-Gruppen und
wobei die kationische Gruppe ein Amin, ein organisch substituiertes Ammonium, ein organisch substituiertes Phosphonium oder einen Metallkomplex umfasst;
B) Kontaktierung der kontaminierten wässrigen Flüssigkeit mit der ersten Adsorbenskomponente wobei die erste Adsorbenskomponente während der Kontaktierung zumindest teilweise in gelöster Form vorliegt, wobei durch die Kontaktierung ein Addukt zwischen der abzutrennenden fluorierten organischen Verbindung und der in gelöster Form vorliegenden ersten Adsorbenskomponente gebildet wird;
D) Abtrennung der zumindest ersten Adsorbenskomponente mit den adsorbierten fluorierten organischen Verbindungen von der wässrigen Flüssigkeit.

2. Verfahren nach dem vorhergehenden Anspruch,
**dadurch gekennzeichnet, dass**
die Abtrennung der fluorierten organischen Verbindungen aus kontaminierten wässrigen Flüssigkeiten mittels eines Kits aus der ersten und einer zweiten Adsorbenskomponente erfolgt, wobei
in Schritt A) zusätzlich die zweite Adsorbenskomponente bereitgestellt wird, wobei die zweite Adsorbenskomponente ein festes Adsorbens ist und wobei das Verfahren ferner folgenden Schritt umfasst:
C) Kontaktierung der wässrigen Flüssigkeit mit der zweiten Adsorbenskomponente.

3. Verfahren nach dem vorhergehenden Anspruch,
**dadurch gekennzeichnet, dass**
Schritt B) und C) gleichzeitig durchgeführt werden oder, dass
Schritt B) vor Schritt C) durchgeführt wird.

4. Verfahren nach einem der vorhergehenden Ansprüche,
**dadurch gekennzeichnet, dass**
die erste Adsorbenskomponente die Formel R¹-E-L-X, R¹-L-E-X oder R¹-E-X besitzt, worin
R¹ die lipophile Gruppe, E die hydrophile Gruppe, L ein Linker und X eine reaktive funktionelle Gruppe ist, die in Schritt B) und/oder Schritt C) eine chemische Reaktion mit der wässrigen Flüssigkeit eingeht oder Wasserstoffbrücken mit dieser ausbilden kann.

5. Verfahren nach dem vorhergehenden Anspruch,
**dadurch gekennzeichnet, dass**
die reaktive Gruppe X eine, zwei oder mehrere Hydroxygruppen aufweist oder durch die Reaktion der Gruppe X mit dem Fluid eine, zwei oder mehrere Hydroxygruppen ausgebildet werden.

6. Verfahren nach dem vorhergehenden Anspruch,
**dadurch gekennzeichnet, dass**
die Gruppe X ausgewählt ist aus Epoxid-Gruppen und Gruppen, die Schritt B) und/oder Schritt C) in einer S_{N}-Reaktion zu einer Hydroxygruppe reagieren, insbesondere 2-Chlorethanol-Gruppen, Alkyl-Sulfonat-Gruppen, Brom-Alkyl-Gruppen oder Iod-Alkylgruppen.

7. Verfahren nach einem der drei vorhergehenden Ansprüche,
**dadurch gekennzeichnet, dass**
in der ersten Adsorbenskomponente der Formel R¹-E-L-X, R¹-L-E-X oder R¹-E-X zwei oder mehr Gruppen X oder X-L an die hydrophile Gruppe gebunden sind und X jeweils eine über einen Methylen-, Ethylen- oder Propylen-Linker gebundene Ether-Gruppe, insbesondere Ethyl- oder Methyl-Ether Gruppe, ist oder dass
in der ersten Adsorbenskomponente der Formel R¹-E-L-X, R¹-L-E-X oder R¹-E-X zumindest eine Gruppe X oder X-L an die hydrophile Gruppe gebunden ist, die eine vicinale Diolgruppe enthält oder einen Precursor für eine durch Reaktion mit der wässrigen Flüssigkeit in Schritt B) und/oder Schritt C) gebildete vicinale Diolgruppe enthält.

8. Verfahren nach einem der vier vorhergehenden Ansprüche,
**dadurch gekennzeichnet, dass**
durch die Reaktion der reaktiven Gruppe X mit der wässrigen Flüssigkeit eine Änderung des pH-Werts der wässrigen Flüssigkeit erfolgt.

9. Verfahren nach dem vorhergehenden Anspruch,
**dadurch gekennzeichnet, dass**
die in Schritt A) bereitgestellte zweite Adsorbenskomponente eine nichtionische Oberfläche aufweist.

10. Verfahren nach einem der vorhergehenden Ansprüche,
**dadurch gekennzeichnet, dass**
die zweite Adsorbenskomponente ausgewählt ist aus Aktivkohlen, Aluminiumoxiden, Kieselgelen, Rußen, Zeolithen und aus faserförmigen oder mikrokristallinen Cellulosen.

11. Verfahren nach einem der vorhergehenden Ansprüche,
**dadurch gekennzeichnet, dass**
vor Schritt D) ein Flockungshilfsmittel zugesetzt wird.

12. Verfahren nach einem der vorhergehenden Ansprüche,
**dadurch gekennzeichnet, dass**
Schritt B) und Schritt C) in einem Rührreaktor oder unter Bedingungen, die denen in einem Rührreaktor entsprechen, durchgeführt werden.

13. Kit zur Abtrennung fluorierter organischer Verbindungen aus kontaminierten wässrigen Flüssigkeiten in einem Verfahren nach einem der vorhergehenden Ansprüche, umfassend eine erste Adsorbenskomponente und eine zweite Adsorbenskomponente, wobei
die zweite Adsorbenskomponente ein festes Adsorbens ist und
die erste Adsorbenskomponente eine chemische Verbindung ist, die eine lipophile Gruppe und eine hydrophile Gruppe enthält, oder eine derartige Verbindung in gelöster Form enthält, wobei die hydrophile Gruppe zumindest eine kationische Gruppe enthält, wobei die lipophile Gruppe ausgewählt ist aus Alkylgruppen, die zumindest eine Octyleneinheit umfassen, aus Aryl-Gruppen und aus Aralkyl-Gruppen und wobei die kationische Gruppe ein Amin, ein organisch substituiertes Ammonium, ein organisch substituiertes Phosphonium oder einen Metallkomplex umfasst und wobei ferner die erste Adsorbenskomponente zumindest teilweise in gelöster Form vorliegt.

14. Kit nach dem vorhergehenden Anspruch, wobei zusätzlich zur ersten und zur zweiten Adsorbenskomponente ein Flockungshilfsmittel enthalten ist.

15. Verwendung einer Verbindung der Formeln R¹-E-L-X, R¹-L-E-X oder der Formel R¹-E-X in zumindest teilweise gelöster Form als erste Adsorbenskomponente zur Abtrennung fluorierter organischer Verbindungen aus kontaminierten wässrigen Flüssigkeiten durch Bildung eines Addukts zwischen der abzutrennenden fluorierten organischen Verbindung und der in gelöster Form vorliegenden ersten Adsorbenskomponente, wobei
R¹ eine lipophile Gruppe ist, die ausgewählt ist aus Alkylgruppen, die zumindest eine Octyleneinheit umfassen, aus Aryl-Gruppen und aus Aralkyl-Gruppen,
E eine hydrophile Gruppe ist, die zumindest eine kationische Gruppe enthält, wobei die kationische Gruppe ein Amin, ein organisch substituiertes Ammonium, ein organisch substituiertes Phosphonium oder einen Metallkomplex umfasst, L ein Linker ist und
X eine reaktive funktionelle Gruppe ist, die eine chemische Reaktion mit der wässrigen Flüssigkeit eingehen kann oder Wasserstoffbrücken mit dieser ausbilden kann.

## Claims

1. A method for the separation of fluorinated organic compounds from contaminated aqueous fluids by means of at least a first adsorbent component comprising the following steps:
A) providing the first adsorbent component, wherein the first adsorbent component is a chemical compound, which contains a lipophilic group and a hydrophilic group, and preferably contains such a compound in dissolved form,
wherein the hydrophilic group contains at least one cationic group,
wherein the lipophilic group is selected from among alkyl groups comprising at least one octylene unit, aryl groups and aralkyl groups, and
wherein the cationic group comprises an amine, an organic substituted ammonium, an organic substituted phosphonium or a metal complex;
B) bringing the contaminated aqueous fluid into contact with the first adsorbent component, wherein the first adsorbent component is present at least partially in dissolved form during the contact, wherein due to the contact an adduct is formed between the fluorinated organic compound to be separated and the first adsorbent component being present in dissolved form;
D) separating the at least first adsorbent component with the adsorbed fluorinated organic from the aqueous fluid.

2. The method according to the preceding claim, **characterized in that** the separation of the fluorinated organic compounds from contaminated aqueous fluids is carried out by means of a kit comprising the first and a second adsorbent component,
wherein the second adsorbent component is additionally provided in step A),
wherein the second adsorbent component is a solid adsorbent and
wherein the method further comprises the following step:
C) bringing the aqueous fluid into contact with the second adsorbent component.

3. The method according to the preceding claim, **characterized in that** steps B) and C) are carried out simultaneously, or that step B) is carried out prior to step C).

4. The method according to one of the preceding claims, **characterized in that** the first adsorbent component has the formula R¹-E-L-X, R¹-L-E-X or R¹-E-X,
wherein
R¹ is a lipophilic group, E is the hydrophilic group, L is a linker and X is a reactive functional group, which chemically reacts with the aqueous fluid in step B) and/or step C), or can form hydrogen bridges therewith.

5. The method according to the preceding claim, **characterized in that**
the reactive group X has one, two or several hydroxy groups, or one, two or several hydroxy groups are formed by the reaction of the group X with the fluid.

6. The method according to the preceding claim, **characterized in that**
the group X is selected from among epoxide groups and groups that react to a hydroxy group in an S_{N} reaction in step B) and/or step C), in particular 2-chloroethanol groups, alkyl sulfonate groups, bromoalkyl groups or iodoalkyl groups.

7. The method according to one of the three preceding claims, **characterized in that**
two or more X or X-L groups are bonded to the hydrophilic group, and that X is an ether group, in particular an ethyl or methyl ether group bonded via a methylene, ethylene or propylene linker in the first adsorbent component having the formula R¹-E-L-X, R¹-L-E-X or R¹-E-X, or that
at least one X or X-L group is bonded to the hydrophilic group, which contains a vicinal diol group or contains a precursor for a vicinal diol group formed by a reaction with the aqueous fluid in step B) and/or step C), in the first adsorbent component having the formula R¹-E-L-X, R¹-L-E-X or R¹-E-X.

8. The method according to one of the four preceding claims, **characterized in that** the pH value of the aqueous fluid is changed as a result of the reaction of the reactive group X with the aqueous fluid.

9. The method according to the preceding claim, **characterized in that**
the second adsorbent component provided in step A) has a non-ionic surface.

10. The method according to one of the preceding claims, **characterized in that** the second adsorbent component is selected from among activated carbon, aluminum oxides, silica gels, soot, zeolites and fibrous or microcrystalline celluloses.

11. The method according to one of the preceding claims, **characterized in that** a flocculant is added prior to step D).

12. The method according to one of the preceding claims, **characterized in that** steps B) and/or step C) are carried out in a stirred tank reactor, or under conditions that correspond to those in a stirred tank reactor.

13. A kit for the separation of fluorinated organic compounds from contaminated aqueous fluids in a method according to the preceding claims,
comprising a first adsorbent component and a second adsorbent component, wherein the second adsorbent component is a solid adsorbent and the first adsorbent component is a chemical compound containing a lipophilic group and a hydrophilic group, or contains such a compound in dissolved form, wherein the hydrophilic group contains at least a cationic group, wherein the lipophilic group is selected from among alkyl groups comprising at least one octylene unit, aryl groups and aralkyl groups, and wherein the cationic group comprises an amine, an organic substituted ammonium, an organic substituted phosphonium, or a metal complex, and wherein the first adsorbent component is furthermore at least partially present in dissolved form.

14. The kit according to the preceding claim, wherein a flocculant is contained in addition to the first and second adsorbent component.

15. The use of a compound having the formula R¹-E-L-X, R¹-L-E-X or the formula R¹-E-X in at least partially present dissolved form as a first adsorbent component for the separation of fluorinated organic compounds from contaminated aqueous fluids by formation of an adduct between the fluorinated organic compound to be separated and the first adsorbent component being present in dissolved form, wherein
R¹ is a lipophilic group selected from among alkyl groups comprising at least one octylene unit, aryl groups and aralkyl groups,
E is a hydrophilic group containing at least one cationic group,
wherein the cationic group comprises an amine, an organic substituted ammonium, an organic substituted phosphonium or a metal complex,
L is a linker, and
X is a reactive group that can react with the fluid, or can form hydrogen bridges therewith.

## Revendications

1. Procédé pour séparer des composés organiques fluorés de liquides aqueux contaminés au moyen d'au moins un premier composant adsorbant comportant les étapes suivantes
A) mise à disposition du premier composant adsorbant, le premier composant adsorbant étant un composé chimique qui contient un groupe lipophile et un groupe hydrophile, et de préférence contient un tel composé sous forme dissoute,
le groupe hydrophile contenant au moins un groupe cationique, le groupe lipophile étant choisi parmi des groupes alkyle, qui comprennent au moins un motif octylène, des groupes aryle et des groupes aralkyle et le groupe cationique comprenant une amine, un ammonium substitué par un groupement organique, un phosphonium substitué par un groupement organique ou un complexe métallique ;
B) mise en contact du liquide aqueux contaminé avec le premier composant adsorbant, le premier composant adsorbant étant présent au moins partiellement sous forme dissoute pendant la mise en contact, un adduit entre le composé organique fluoré devant être séparé et le premier composant adsorbant présent sous forme dissoute étant formé par la mise en contact ;
D) séparation de l'au moins un premier composant adsorbant comportant les composés organiques fluorés adsorbés du liquide aqueux.

2. Procédé selon la revendication précédente, **caractérisé en ce que** la séparation des composés organiques fluorés et des liquides aqueux contaminés est réalisée au moyen d'un kit composé du premier et du deuxième composant adsorbant,
de plus dans l'étape A) le deuxième composant adsorbant étant mis à disposition, le deuxième composant adsorbant étant un adsorbant solide et le procédé comprenant en outre l'étape suivante :
C) mise en contact du liquide aqueux avec le deuxième composant adsorbant.

3. Procédé selon la revendication précédente, **caractérisé en ce que** les étapes B) et C) sont mises en œuvre simultanément ou, **en ce que** l'étape B) est mise en œuvre avant l'étape C).

4. Procédé selon l'une quelconque des revendications précédentes, **caractérisé en ce que** le premier composant adsorbant possède la formule R¹-E-L-X, R¹-L-E-X ou R¹-E-X,
R¹ étant le groupe lipophile, E étant le groupe hydrophile, L étant un lieur et X étant un groupe fonctionnel réactif, qui, dans l'étape B) et/ou l'étape C), entre en réaction chimique avec le liquide aqueux ou qui peut former des liaisons hydrogène avec celui-ci.

5. Procédé selon la revendication précédente, **caractérisé en ce que** le groupe réactif X présente un, deux ou plusieurs groupes hydroxy ou par la réaction du groupe X avec le fluide, un, deux ou plusieurs groupes hydroxy sont formés.

6. Procédé selon la revendication précédente, **caractérisé en ce que** le groupe X est choisi parmi des groupes époxyde et des groupes qui réagissent dans l'étape B) et/ou l'étape C) dans une réaction S_{N} pour donner un groupe hydroxy, en particulier des groupes 2-chloroéthanol, des groupes alkylsulfonate, des groupes bromoalkyle ou des groupes iodoalkyle.

7. Procédé selon l'une des trois revendications précédentes, **caractérisé en ce que** dans le premier composant adsorbant possédant la formule R¹-E-L-X, R¹-L-E-X ou R¹-E-X, deux groupes X ou X-L ou plus sont liés au groupe hydrophile et X est à chaque fois un groupe éther lié par l'intermédiaire d'un lieur méthylène, éthylène ou propylène, en particulier un groupe éthyléther ou méthyléther, ou **en ce que** dans le premier composant adsorbant possédant la formule R¹-E-L-X, R¹-L-E-X ou R¹-E-X, au moins un groupe X ou X-L est lié au groupe hydrophile, qui contient un groupe diol vicinal ou contient un précurseur pour un groupe diol vicinal formé par réaction avec le liquide aqueux dans l'étape B) et/ou l'étape C).

8. Procédé selon l'une des quatre revendications précédentes, **caractérisé en ce que** par la réaction du groupe réactif X avec le liquide aqueux, une modification de la valeur de pH du liquide aqueux a lieu.

9. Procédé selon la revendication précédente, **caractérisé en ce que** le deuxième composant adsorbant mis à disposition dans l'étape A) présente une surface non ionique.

10. Procédé selon l'une quelconque des revendications précédentes, **caractérisé en ce que** le deuxième composant adsorbant est choisi parmi les charbons actifs, les oxydes d'aluminium, les gels de silice, les suies, les zéolithes et parmi les celluloses fibreuses ou microcristallines.

11. Procédé selon l'une quelconque des revendications précédentes, **caractérisé en ce qu'**avant l'étape D), un auxiliaire de floculation est ajouté.

12. Procédé selon l'une quelconque des revendications précédentes, **caractérisé en ce que** l'étape B) et l'étape C) sont mises en œuvre dans un réacteur d'agitation ou dans des conditions qui correspondent à celles dans un réacteur d'agitation.

13. Kit pour séparer des composés organiques fluorés de liquides aqueux contaminés dans un procédé selon l'une quelconque des revendications précédentes, comprenant un premier composant adsorbant et un deuxième composant adsorbant,
le deuxième composant adsorbant étant un adsorbant solide et le premier composant adsorbant étant un composé chimique qui contient un groupe lipophile et un groupe hydrophile, ou contient un tel composé sous forme dissoute, le groupe hydrophile contenant au moins un groupe cationique, le groupe lipophile étant choisi parmi des groupes alkyle, qui comprennent au moins un motif octylène, des groupes aryle et des groupes aralkyle et le groupe cationique comprenant une amine, un ammonium substitué par un groupement organique, un phosphonium substitué par un groupement organique ou un complexe métallique et en outre le premier composant adsorbant étant présent au moins partiellement sous forme dissoute.

14. Kit selon la revendication précédente, où en plus du premier et du deuxième composant adsorbant, un auxiliaire de floculation est contenu.

15. Utilisation d'un composé des formules R¹-E-L-X, R¹-L-E-X ou de formule R¹-E-X sous forme au moins partiellement dissoute en tant que premier composant adsorbant pour la séparation de composés organiques fluorés de liquides aqueux contaminés par formation d'un adduit entre le composé organique fluoré devant être séparé et le premier composant adsorbant présent sous forme dissoute,
R¹ étant un groupe lipophile, qui est choisi parmi des groupes alkyle, qui comprennent au moins un motif octylène, des groupes aryle et des groupes aralkyle,
E étant un groupe hydrophile, qui contient au moins un groupe cationique, le groupe cationique comprenant une amine, un ammonium substitué par un groupement organique, un phosphonium substitué par un groupement organique ou un complexe métallique, L étant un lieur et
X étant un groupe fonctionnel réactif, qui peut entrer en réaction chimique avec le liquide aqueux ou former des liaisons hydrogène avec celui-ci.
